# EUROPÄISCHE PATENTSCHRIFT

(11) **EP 0 913 154 B1**
(45) Veröffentlichungstag und Bekanntmachung des Hinweises auf die Patenterteilung: **20.11.2002**
(21) Anmeldenummer: 98115049.3
(22) Anmeldetag: 11.08.1998
(51) Int. Cl.: A61K 31/57, A61K 31/58, A61K 9/70, A61K 7/043

(54) **Antipsoriatisch wirksamer Nagellack**
Antipsoriatic nail lacquer
Vernis à ongles antipsoriatique

(30) Priorität: 21.08.1997 DE 19736112
(43) Veröffentlichungstag der Anmeldung: 06.05.1999
(73) Patentinhaber: Aventis Pharma Deutschland GmbH, 65929 Frankfurt am Main (DE)
(72) Erfinder: Bohn, Manfred, Dr., 65719 Hofheim (DE); Kraemer, Karl Theodor, Dr., 63225 Langen (DE)

(56) Entgegenhaltungen:
- EP-A1- 0 226 984
- EP-A2- 0 515 312
- WO-A1-87/02580
- WO-A1-95/03838
- WO-A1-97/34644
- DE-A- 2 423 849
- DE-A1- 19 529 085
- GB-A- 2 188 844
- US-A- 3 861 932
- US-A- 4 210 633
- US-A- 4 250 164
- Database WPIL on Questel, Woche 8504, London: Derwent Publications Ltd., AN 85-021807, Klasse A61K, XP002900243; & JP 59-216822 A (TERUMO CORP)

## Beschreibung

Die Beteiligung der Finger- und Fußnägel am Krankheitsbild der Psoriasis ist weit verbreitet. Literaturangaben zufolge zeigen bis zu 50 % aller Psoriasis Patienten neben den charakteristischen Hautsymptomen zusätzlich Veränderungen der Nägel. Die Nagelveränderungen finden sich an den Fingern häufiger als an den Zehen und sind in der Reihenfolge ihrer Häufigkeit durch folgende Symptome gekennzeichnet:

Grübchenbildung (in einem bestimmten Muster oder auch unregelmäßig auf der Nagelplattenoberfläche angeordnete, punktförmige oder unregelmäßig geformte Vertiefungen), Verfärbung des Nagelbettes, Onycholyse (Ablösung der Nagelplatte vom Nagelbett), subunguale Keratose oder Anomalien der Nagelplatte.

Zur Behandlung der von Psoriasis befallenen Nägel kamen bislang, allerdings ohne durchschlagenden Erfolg, neben der PUVA-Lichttherapie folgende vier Methoden zur Anwendung:

Eine Behandlungsmethode, die systemische, besteht darin, daß man Methotrexat, Retinoide oder Cyclosporin A oral verabreicht. Dies erfordert eine Langzeitbehandlung, die erfahrungsgemäß zu Intoxikationen führen kann.

Eine andere Methode besteht darin, intraläsional Kortikosteroide zu injizieren. Diese Methode ist naturgemäß sehr schmerzhaft, so daß die Patienten nur anfangs bereit sind mitzuarbeiten, später der Behandlung aber fernbleiben.

Mit einer weiteren Methode, die befallenen Nägel chirurgisch zu entfernen, lassen sich zwar gute Behandlungsergebnisse erzielen, jedoch ist dabei zu bedenken, ob sich der Eingriff bei einer möglichen Rezidivzeit von einer Woche nach Neubildung der Nagelplate wirklich lohnt.

Eine vierte, aber schonende Methode besteht darin, daß man die Nägel lokal mit spezifischen, antipsoriatisch wirksamen Stoffen wie Dithranol, Vitamin D Analoga oder Kortikosteroiden behandelt. Hierbei hat man die unterschiedlichsten Behandlungsmethoden versucht. So hat man in einer kombinierten Behandlung die Nägel zunächst mit Lösungen der antipsoriatisch wirksamen Stoffe behandelt und nachts jeweils Cremeverbände angelegt. Auch diese Behandlungsmethode ist für den Patienten naturgemäß sehr unerfreulich und psychisch belastend. Einmal ist die Behandlung der Nägel mit Lösungen mehrmals täglich erforderlich. Zum anderen müssen die behandelten Nägel nachts mit Verbänden abgedeckt werden.

Dies führt dazu, daß die gewöhnlich vielmonatige Behandlung von den Patienten vielfach nicht durchgehalten wird, sie vielmehr entmutigt und nachlässig werden und daß damit der Therapieerfolg ausbleibt. Der Behandlungserfolg wird bei dieser Methode weiterhin dadurch beeinträchtigt, daß die Lösungen und Cremes gewöhnlich mit Wasser mischbar bzw. hydrophil sind und daher beim Waschen, Baden und Duschen von der Nageloberfläche wieder entfernt bzw. aus dem Nagel herausgelöst werden können, mithin also danach wieder neu aufgetragen werden müssen. Die Behandlung mit diesen Topika ist infolgedessen ineffektiv und zudem höchst unökonomisch.

Man hat daher große Hoffnungen in eine andere Methode gesetzt, nämlich in die Behandlung der befallenen Nägel mit einer 50:50 Mischung aus handelsüblichen Kortikosteroid-haltigen Lotionen, Cremes oder Salben mit handelsüblichen kosmetischen Klarlacken (US Patent 4,250,164). Diese Methode hat jedoch, obwohl schon lange bekannt, keinen allgemeinen Eingang in die Therapie gefunden, da sich ein zufriedenstellender Erfolg dieser - naturgemäß physikalisch instabilen - Mischungen, vermutlich mangels ausreichender Bioverfügbarkeit des Wirkstoffes aus dem nach dem Eintrocknen der Mischung vorliegenden Feststoffsystem nicht einstellte.

Daher hat man viele Fälle, insbesondere die schweren, nach wie vor mit der oben beschriebenen chirurgischen Methode oder mit intraläsionalen Injektionen bzw. mit der kombinierten Lösungs- und Cremetherapie behandelt.

WO 97/34 644 beschreibt eine topische Formulierung enthaltend einen antipsoriatischen Wirkstoff, mindestens ein spreitendes Lösungsmittel, mindestens ein flüchtiges Lösungsmittel und einen Filmbildner zur Behandlung von Nagelpsoriasis. Diese Zubereitung (Nagellack) hat den Nachteil, daß ein spreitendes Lösungsmittel benötigt wird, das zusätzliche Kosten bei der Herstellung verursacht und dessen pharmakologische Verträglichkeit zweifelhaft ist und somit ebenfalls die Arzneimittelzulassung kompliziert bzw. unmöglich macht.

Die Erfindung bezweckt eine glukokortikoidhaltige Formulierung zur Verfügung zu stellen, die vorstehend beschriebene Nachteile nicht oder nur in untergeordnetem Maße besitzt. So sollte die Formulierung eine gute Penetration des Nagels durch das Glukokortikoid und somit eine gute Bioverfügbarkeit gewährleisten und dies bei Verwendung eines wasserlöslichen Filmbildners.

Ganz besonders erwünscht war es eine Formulierung zu finden, dia auch ohne zusätzliche Spreit- bzw. Penetrationsförderer eine gute Penetration des Nagels durch das Glukokortikoid und somit eine gute Bioverfügbarkeit gewährleistet.

Die Aufgabe wird gelöst durch den erfindungsgemäßen Nagellack bestehend aus einem oder mehreren Glukokortikoiden, in einer Gesamtkonzentration von 0,5 % bis 20 %, bezogen auf das Gesamtgewicht der Formulierung, einem oder mehreren wasserunlöslichen Filmbildnern und einem oder mehreren physiologische unbedenklichen Lösungsmitteln, wobei spreitende Lösungsmittel ausgenommen sind.

Antipsoriatisch wirksame Glukokortikoide sind z.B.: Alclometasondipropionat, Amcinonid, Beclomethasondipropionat, Bendacort, Betamethasonbenzoat, Betamethasondipropionat, Betamethasonvalerat, Budesonid, Chlorquinaldol, Clioquinol, Clobetasolpropionat, Clobetasonbutyrat, Desonid, Desoximetason, Dexamethason, Dichlorison, Diflorasondiacetat, Diflucortolonvalerat, Difluprednat, Fluazacort, Flucinolonacetonid, Fluclorolonacetonid, Fludroxycortid, Flumathasonpivalat, Fluocinolonacetonide, Fluocinoid, Fluocortolon, Fluormetholon, Flupameson, Flupredniden, Fluprednidenacetat, Flurandrenolid, Halcinonid, Halometason, Hydrocortamat, Hydrocortisonbutyrat, Methylprednisolonaceponat, Mometasonfuroat, Prednicarbat, Prednisolon, Prednison, Tixocortol, Triamcinolonacetonid.

Die Glukokortikosteroide können sowohl als freie Alkohole als auch in Form ihrer Ester zugegen sein.

Als wasserunlösliche Filmbildner eignen sich z.B. Stoffe auf Cellulosebasis wie Cellulosenitrat oder Ethylcellulose oder physiologisch unbedenkliche Polymerisate, wie sie z.B. in Kosmetika üblich sind. Genannt seien beispielsweise Polyvinylacetat und partiell verseiftes Polyvinylacetat, Mischpolymerisate aus Vinylacetat einerseits und Acrylsäure oder Crotonsäure oder Maleinsäuremonoalkylester andererseits, ternäre Mischpolymerisate aus Vinylacetat einerseits und Crotonsäure und Vinylneodecanoat, oder Crotonsäure und Vinylpropionat andererseits, Mischpolymerisate aus Methylvinylether und Maleinsäuremonoalkylester, insbesondere als Maleinsäuremonobutylester, Mischpolymerisate aus Fettsäurevinylester und Acrylsäure oder Methacrylsäure, Mischpolymerisate aus N-Vinylpyrrolidon, Methacrylsäure und Methacrylsäurealkylester, Mischpolymerisate aus Acrylsäure und Methacrylsäure oder Acrylsäurealkylester oder Methacrylsäurealkylester, insbesondere mit einem Gehalt an quartären Ammoniumgruppen, oder Polymere, Copolymere oder Mischungen, enthaltend Ethylacrylat, Methylmethacrylat oder Trimethylammonioethylmethacrylat-chlorid, oder Polyvinylacetale und Polyvinylbutyrale, alkylsubstituierte Poly-N-vinylpyrrolidone, Alkylester aus Mischpolymerisaten aus Olefinen und Maleinsäureanhydrid, Umsetzungsprodukte von Kolophonium mit Acrylsäure sowie Benzoeharze. In den Estern sind die Alkylreste gewöhnlich kurzkettig und haben meistens nicht mehr als vier C-Atome.

Als physiologisch unbedenkliche Lösemittel kommen Stoffe wie in Kosmetika übliche Kohlenwasserstoffe, halogenierte Kohlenwasserstoffe, Alkohole, Äther, Ketone und Ester in Betracht, insbesondere Essigsäureester von einwertigen Alkoholen wie Ethyl- und Butylacetat, gegebenenfalls im Gemisch mit aromatischen Kohlenwasserstoffen wie Toluo und/oder Alkoholen wie Ethanol oder lsopropanol.

Die Kombination der Lösemittel ist bekanntlich von entscheidender Bedeutung für die Trockenzeit, Verstreichbarkeit und andere wichtige Eigenschaften des Lackes bzw. des Lackfilms. Das Lösemittelsystem besteht vorzugsweise aus einer optimalen Mischung aus Niedrigsiedern (= Lösemittel mit einem Siedepunkt bis 100°C) und Mittelsiedern (= Lösemittel mit einem Siedepunkt bis 150°C), gegebenenfalls mit einem kleinen Anteil Hochsiedem (= Lösemittel mit einem Siedepunkt bis 200°C).

Unter leicht flüchtigen Lösungsmitteln werden Verbindungen verstanden, die einen Siedepunkt besitzen, der unter 80°C liegt.

Die erfindungsgemäßen Nagellacke können weiterhin in Kosmetika gebräuchliche Zusätze enthalten, wie Weichmacher auf Phthalat- oder Campherbasis, Farbstoffe bzw. Farbpigmente, Perlglanzmittel, Sedimentationsverzögerer, Sulfonamidharze, Silikate, Riechstoffe, Lanolinderivate, Lichtschutzmittel wie 2-Hydroxy-4-methoxybenzophenon, antimikrobiell wirksame Substanzen und Stoffe mit keratolytischer und/oder keratoplastischer Wirkung, wie Ammoniumsulfit, Ester und Salze der Thioglykolsäure, Harnstoff, Allantoin, Enzyme und Salizylsäure.

Mit dem erfindungsgemäßen Nagellack läßt sich bei der Behandlung von psoriatischen Nägeln eine durchgreifende Heilung erzielen, wobei der Nagel gewöhnlich ohne Deformierung nachwächst. Im Hinblick auf die bisherigen schlechten Therapieerfahrungen ist dies ein überaus wichtiger Befund.

Der erfindungsgemäße Nagellack eignet sich auch zur prophylaktischen Anwendung gegen psoriatische Nägel, wobei ein ausreichend hohes Wirkstoffdepot im Nagel erreicht wird, so daß ein mögliches Rezidiv nicht zum Ausbruch kommt.

Der Gehalt an Wirkstoff in dem erfindungsgemäßen Nagellack ist von der Struktur eines jeden Wirkstoffes und damit von dessen Freigabe aus dem Lackfilm, seinem Penetrationsverhalten im Nagel und seiner Wirkstärke abhängig.

In dem erfindungsgemäßen Nagellack, also der Lösemittel enthaltenden Anwendungsform, ist der Wirkstoff im allgemeinen in einer Menge von 0,5 bis 20, vorzugsweise 2 bis 15 Gewichtsprozent enthalten. Der Mindestgehalt an Wirkstoff in den medizinischen Nagellacken, also denen zur Behandlung, beträgt vorzugsweise 8 Gewichtsprozent; die zur Prophylaxe verwendeten Nagellacke enthalten vorzugsweise 1 bis 4 Gewichtsprozent Gewichtsprozent Wirkstoff.

Gefärbte bzw. pigmentierte Nagellacke haben z.B. den Vorteil, das die erfindungsgemäße Zubereitung dem Schönheitsempfinden des Patienten angepaßt werden kann.

Die Herstellung des Nagellackes erfolgt in üblicher Weise durch Zusammengeben der einzelnen Komponenten und eine - soweit erforderlich - der jeweiligen Zubereitung angepaßte Weiterverarbeitung.

Der erfindungsgemäße Nagellack weist folgende Zusammensetzung auf:

### Beispiel 1

| | |
|---|---|
| Clobetasol-17-propionat | 8,0 % |
| 50 %ige Lösung eines Mischpolymerisates aus Methylvinyläther und Maleinsäuremonobutylester in Isopropylalkohol | 30,0 % |
| Isopropylalkohol | 31,0 % |
| Äthylacetat | 31,0 % |

Die prozentualen Mengenangaben sind auf das Gewicht bezogen.

Der Nagellack wird durch Lösen der verschiedenen Komponenten in den Lösemitteln hergestellt.

### Beispiel 2

| | |
|---|---|
| Desoximetason | 5,0 % |
| Ethylacrylat-Methylmethacrylat-Trimethylammonioethyl-methacrylat-chlorid in einem Molverhältnis von 1:2:0,2 (EUDRAGIT® RL 100) | 12,0 % |
| Ethanol 96 % | 60,0 % |
| Ethylacetat | 13,0 % |
| Butylacetat | 10,0 % |

### Beispiel 3

| | |
|---|---|
| Betamethasondipropionat | 5,0 % |
| Ethylcellulose | 11,0 % |
| Ethylacetat | 30,0 % |
| Butylacetat | 34,0 % |
| Ethanol 96 % | 20,0 % |

### Beispiel 4

| | |
|---|---|
| Prednicarbat | 7,5 % |
| Polyvinylbutyral | 4,7 % |
| Cellulosenitrat | 4,3 % |
| Dibutylphthalat | 0,6 % |
| Ethylacetat | 10,0 % |
| Ethanol 96 % | 72,9 % |

### Beispiel 5

| | |
|---|---|
| Halcinonid | 2,0 % |
| Methacrylsäure-Ethylacrylat - 1:1 Copolymer | 6,5 % |
| Ethanol 96 % | 71,5 % |
| Ethylacetat | 20,0 % |

Die Wirkung des erfindungsgemäßen Nagellacks wird in Permeationstests an Kuhhornplättchen und in Behandlungsversuchen an Probanden nachgewiesen. Der Permeationstest an Kuhhornplättchen erlaubt es, die Freigabe eines Wirkstoffes aus einer bestimmten Zubereitung und die sich daran anschließende Permeation durch Keratinmaterial zu prüfen.

Derzeit sind noch keine topische Zubereitungen zur Behandlung der Nagelpsoriasis mit Glukokortikoiden bekannt, aus denen der Wirkstoff in ausreichender Menge freigegeben wird, in die Nägel eindringt und somit in einer therapeutischen Dosis auf die darunterliegende Matrix oder das Nagelbett einwirken kann.

### Als Kontrollbeispiel wird

| | |
|---|---|
| Clobetasol-17-propionat | 8,0 % |
| in Isopropylalkohol | 92,0 % |

gelöst.

### A) Permeationstest an Kuhhornplättchen

Die Messung der Wirkstoffpermeation wird mittels zeitaufgelöster ATR Technik (Time resolved infrared attenuated total reflection - siehe Th. M. Bayerl et al.; J. Invest. Dermatol. 105:291-295,1995) durchgeführt:

Auf die Oberseite eines Kuhhornplättchens von 0,5 mm Dicke werden auf einer definierten Fläche 100 µl der Prüfzubereitung (Prüfzubereitung oder Kontrollbeispiel) aufgetragen. Nach einer Trocknungszeit von 15 Minuten wurde das Kuhhornplättchen mit der Lackschicht auf den Meßkristall aufgebracht und durch eine externe Vorrichtung angedrückt. Der Anpreßdruck und die Eindringtiefe des Meßstrahles wurde dabei so gewählt, daß das IR-Spektrum keine Anteile des Kuhhornplättchens erfaßte. 48 Stunden lang wurden Spektren der Lackschicht aufgenommen und die Abnahme der Wirkstoffbanden, die auf das Eindringen des Wirkstoffes in das Keratinmaterial zurückzuführen ist, untersucht.

Dabei zeigte sich, daß die charakteristische Bande von Clobetasol-17-propionat bei 1660 cm⁻¹ in dem klaren Lackfilm des erfindungsgemäßen Nagellacks im Meßzeitraum von 48 Stunden auf etwa 60 % des Ausgangswertes abnimmt, während der Wirkstoff aus dem Kontrollbeispiel beim Abdunsten des Lösemittels nahezu quantitativ ausfällt und somit für ein Eindringen in das Keratinmaterial nicht mehr zur Verfügung steht.

Darüberhinaus konnte auf der Rückseite der eingesetzten Kuhhornplättchen nach dem Auftragen des erfindungsgemäßen Nagellacks Clobetasol-17-propionat qualitativ nachgewiesen werden, während nach dem Auftragen der Kontrollzubereitung dieser Nachweis nicht erbracht werden konnte.

Obwohl in EP 0 226 984 ähnliche Penetrationseigenschaften von bestimmten antimykotisch wirksamen Hydroxypyridonverbindungen aus festen Lackfilmen beschrieben werden, ist dies dennoch ein überraschender Befund, da nicht vorauszusehen war, daß Glukokorticoide, die ein sperriges, starres Cyclopentanoperhydrophenanthren Vierringsystem darstellen, aus dem nach dem Trocknen der Lackzubereitung vorliegenden wasserunlöslichen Feststoffsystem, besser bioverfügbar sind und in bzw. durch das Keratinmaterial permeieren als aus der isopropanolischen Lösung.

### B) Wirksamkeitsprüfung

1) Die antipsoriatisch wirksamen Eigenschaften des erfindungsgemäßen Nagellacks ist an 2 Menschen mit langjähriger beidhändiger Daumennagelpsoriasis überprüft worden. Eine nur viermonatige tägliche Behandlung der befallenen Nägel mit dem erfindungsgemäßen Nagellack gemäß Beispiel 1 führte zu dem Herauswachsen von symptomfreien neuen Nagelplatten.
2) In einem weiteren Therapieversuch an 14 Patienten mit Nagelpsoriasis wurde gezeigt, daß bei zweimal wöchentlicher Anwendung mit dem erfindungsgemäßen Nagellack gemäß Beispiel 1 nach 6 Monaten in 86 % der Fälle eine nachhaltige Besserung oder sogar vollständige Symptomfreiheit erzielt werden konnte.

## Patentansprüche

1. Nagellack bestehend aus einem oder mehreren Glukokortikoiden, in einer Gesamtkonzentration von 0,5 % bis 20 %, bezogen auf das Gesamtgewicht der Formulierung, einem oder mehreren wasserunlöslichen Filmbildnern und einem oder mehreren physiologisch unbedenklichen Lösungsmitteln, wobei spreitende Lösungsmittel ausgenommen sind.

2. Nagellack gemäß Anspruch 1, **dadurch gekennzeichnet, daß** er als Glukokortikoid, Alclometasondipropionat, Amcinonid, Beclomethasondipropionat, Bendacort, Betamethasonbenzoat, Betamethasondipropionat, Betamethasonvalerat, Budesonid, Chlorquinaldol, Clioquinol, Clobetasolpropionat, Clobetasonbutyrat, Desonid, Desoximetason, Dexamethason, Dichlorison, Diflorasondiacetat, Diflucortolonvalerat, Difluprednat, Fluazacort, Flucinolonacetonid, Fluclorolonacetonid, Fludroxycortid, Flumathasonpivalat, Fluocinolonacetonide, Fluocinoid, Fluocortolon, Fluormetholon, Flupameson, Flupredniden, Fluprednidenacetat, Flurandrenolid, Halcinonid, Halometason, Hydrocortamat, Hydrocortisonbutyrat, Methylprednisolonaceponat, Mometasonfuroat, Prednicarbat, Prednisolon, Prednison, Tixocortol oder Triamcinolonacetonid enthält.

3. Nagellack gemäß einem oder mehreren der Ansprüche 1 und 2, **dadurch gekennzeichnet, daß** er als Wirkstoff Clobetasolpropionat, Desoximetason, Betamethasondipropionat, Prednicarbat oder Halcinonid enthält.

4. Nagellack gemäß einem oder mehreren der Ansprüche 1 bis 3, **dadurch gekennzeichnet, daß** er den Wirkstoff bzw. die Wirkstoffe in einer Gesamtkonzentration von 2 % bis 15 %, bezogen auf das Gesamtgewicht der Formulierung enthält.

5. Nagellack gemäß einem oder mehreren der Ansprüche 1 bis 4, **dadurch gekennzeichnet, daß** er als wasserunlöslicher Filmbildner ein Mischpolymerisat aus Methylvinyläther und Maleinsäuremonobutylester; Ethylacrylat-Methylmethacrylat-Trimethylammonioethyl-methacrylat-chlorid in einem Molverhältnis von 1:2:0,2; Ethylcellulose; Polyvinylbutyral, Cellulosenitrat oder Methacrylsäure-Ethylacrylat Copolymer enthält.

6. Verfahren zur Herstellung eines Nagellacks gemäß einem oder mehreren der Ansprüche 1 bis 5, **dadurch gekennzeichnet, daß** man einen wasserunlöslichen Filmbildner mit einem Glukokortikoid, sowie ggf. anderen für die Herstellung von Nagellack üblichen Komponenten vermischt.

## Claims

1. A nail lacquer consisting of one or more glucocorticoids, in a total concentration of 0.5% to 20%, based on the total weight of the formulation, one or more water-insoluble film-forming agents and one or more physiologically acceptable solvents, spreading solvents being excepted.

2. The nail lacquer as claimed in claim 1, **characterized in that** as glucocorticoid it contains alclometasone dipropionate, amcinonide, beclomethasone dipropionate, bendacort, betamethasone benzoate, betamethasone dipropionate, betamethasone valerate, budesonide, chlorquinaldol, clioquinol, clobetasol propionate, clobetasone butyrate, desonide, desoxymetasone, dexamethasone, dichlorisone, diflorasone diacetate, diflucortolone valerate, difluprednate, fluazacort, flucinolone acetonide, fluclorolone acetonide, fludroxycortide, flumethasone pivalate, fluocinolone acetonide, fluocinoid, fluocortolone, fluormetholone, flupamesone, fluprednidene, fluprednidene acetate, flurandrenolide, halcinonide, halometasone, hydrocortamate, hydrocortisone butyrate, methylprednisolone aceponate, mometasone furoate, prednicarbate, prednisolone, prednisone, tixocortol or triamcinolone acetonide.

3. The nail lacquer as claimed in one or more of claims 1 and 2, **characterized in that** as active compound it contains clobetasol propionate, desoxymetasone, betamethasone dipropionate, prednicarbate or halcinonide.

4. The nail lacquer as claimed in one or more of claims 1 to 3, **characterized in that** it contains the active compound or the active compounds in a total concentration of 2% to 15%, based on the total weight of the formulation.

5. The nail lacquer as claimed in one or more of. claims 1 to 4, **characterized in that** it as water-insoluble film-forming agent contains a copolymer of methyl vinyl ether and monobutyl maleate; ethyl acrylate-methyl methacrylate-trimethylammonioethyl methacrylate chloride in a molar ratio of 1:2:0.2; ethylcellulose; polyvinylbutyral, cellulose nitrate or methacrylic acid-ethyl acrylate copolymer.

6. A process for the production of a nail lacquer as claimed in one or more of claims 1 to 5, **characterized in that** it comprises mixing a water-insoluble film-forming agent with a glucocorticoid, and optionally other components customary for the production of nail lacquer.

## Revendications

1. Vernis à ongles composé d'un ou plusieurs glucocorticoïdes, dans une concentration totale de 0,5% à 20%, par rapport au poids total de la formulation, d'un ou plusieurs agents filmogènes insolubles dans l'eau et d'un ou plusieurs solvants physiologiquement neutres, parmi lesquels on fait exception des diluants.

2. Vernis à ongles selon la revendication 1, **caractérisé en ce qu'**il contient comme glucocorticoïde le dipropionate d'alclométasone, l'amcinonide, le dipropionate de béclométhasone, le bendacort, le benzoate de bétaméthasone, le dipropionate de bétaméthasone, le valérate de bétaméthasone, le budésonide, le chlorquinaldol, le clioquinol, le propionate de clobétasol, le butyrate de clobétasone, le désonide, la désoxymétasone, la déxaméthasone, la dichlorisone, le diacétate de diflorasone, le valérate de diflucortolone, le difluprednate, le fluazacort, l'acétonide de flucinolone, l'acétonide de fluclorolone, le fludroxycortide, le pivalate de fluméthasone, l'acétonide de fluocinolone, le fluocinoide, la fluocortolone, la fluorométholone, la flupamésone, le fluprednidène, l'acétate de fluprednidène, le flurandrénolide, l'halcinonide, l'halométasone, l'hydrocortamate, le butyrate d'hydrocortisone, l'acéponate de méthylprednisolone, le furoate de mométasone, le prednicarbate, la prednisolone, la prednisone, le tixocortol ou l'acétonide de triamcinolone.

3. Vernis à ongles selon une ou plusieurs des revendications 1 et 2, **caractérisé en ce qu'**il contient comme principe actif le propionate de clobétasol, la désoxymétasone, le dipropionate de bétaméthasone, le prednicarbate ou l'halcinonide.

4. Vernis à ongles selon une ou plusieurs des revendications 1 à 3, **caractérisé en ce qu'**il contient le principe actif ou les principes actifs dans une concentration en poids de 2% à 15% par rapport au poids total de la formulation.

5. Vernis à ongles selon une ou plusieurs des revendications 1 à 4, **caractérisé en ce qu'**il contient comme agent filmogène insoluble dans l'eau un copolymère d'éther méthylvinylique et de maléate de butyle; le chlorure d'acrylate d'éthyle-méthacrylate de méthyle-méthacrylate de triméthylammonioéthyle dans un rapport molaire de 1:2:0,2; l'éthylcellulose; le polyvinylbutyral, le nitrate de cellulose ou un copolymère d'acide méthacrylique-éthylacrylate.

6. Procédé pour la préparation d'un vernis à ongles selon une ou plusieurs des revendications 1 à 5, **caractérisé en ce que** l'on mélange un agent filmogène insoluble dans l'eau avec un glucocorticoïde, ainsi que, le cas échéant, d'autres composants courants pour la préparation de vernis à ongles.
